# EUROPEAN PATENT SPECIFICATION

(11) **EP 4 279 040 B1**
(45) Date of publication and mention of the grant of the patent: **23.07.2025**
(21) Application number: 23753777.4
(22) Date of filing: 23.03.2023
(51) Int. Cl.: A61F 5/05, A61F 5/01, A44B 11/00, A41D 13/06, A41F 1/00, A44B 11/25

(54) **MAGNETIC BUCKLE STRUCTURE FOR KNEE JOINT BRACE, AND KNEE JOINT BRACE**
MAGNETISCHE SCHNALLENSTRUKTUR FÜR EINE KNIEGELENKSORTHESE UND KNIEGELENKSORTHESE
STRUCTURE DE BOUCLE MAGNÉTIQUE POUR ATTELLE D'ARTICULATION DU GENOU, ET ATTELLE D'ARTICULATION DU GENOU

(30) Priority: 25.03.2022 CN 202210300786
(43) Date of publication of application: 22.11.2023
(73) Proprietor: Vincent Medical (Dong Guan) Manufacturing Co., Ltd., Dongguan, Guangdong 523730 (CN); Vincent Medical Manufactuing Co., Ltd., Hung Hom, Hong Kong (HK); Rehab-Robotics Company Limited, Kowloon, Hong Kong (HK)
(72) Inventor: XIA, Mingjun, Guangdong 523730 (CN); LIANG, Wei, Guangdong 523730 (CN); FU, Guofu, Guangdong 523730 (CN); CAI, Jialin, Guangdong 523730 (CN); XU, Peiyong, Guangdong 523730 (CN); CHEN, Yongjian, Guangdong 523730 (CN)
(74) Representative: KIPA AB
(86) International application number: PCT/CN2023/083439
(87) International publication number: WO 2023/179722

(56) References cited:
- CN-A- 102 481 034
- CN-A- 109 549 766
- CN-A- 113 580 101
- CN-A- 114 869 700
- CN-U- 207 429 243
- CN-U- 207 492 844
- CN-U- 213 430 890
- KR-A- 20090 069 288
- KR-A- 20110 026 543
- KR-A- 20190 058 083
- TW-U- 554 683
- US-A1- 2010 130 899
- US-A1- 2011 168 485
- US-A1- 2013 097 824
- US-A1- 2020 069 002
- US-B2- 9 687 376

## Description

### TECHNICAL FIELD

The present disclosure relates to the technical field of rehabilitation and sports equipment, more particularly to a magnetic buckle structure for a knee-joint brace and a knee-joint brace having the same.

### BACKGROUND

Knee joint is one of the j oints in the human body that has a high load-bearing capacity and a complex structure. Knee joint diseases often occur in daily life, leading to limited human movement and seriously reducing the quality of life. In order to alleviate the damage of the knee joint diseases to the human body and improve the quality of life, a knee-joint brace is provided in the existing technologies, with which a support structure (such as a support arm) is arranged on the periphery of the knee joint to assist in supporting the knee joint associated bones, whereby assisting in fixing and supporting the knee joint. Therefore, the knee-joint brace can provide great protection and support for those people with knee joint discomfort, can reduce the pain and discomfort of the knee joints, and can provide walking support to certain extent for those people with walking disabilities, thereby reducing the condition of limited human movement and improving the quality of their lives.

However, for most of those patients with knee-joint diseases, their self-care abilities are greatly limited, and they often have problems in wearing and removing the knee-joint braces.

US 2011/0168485 A1 discloses a belt buckle device including a tongue piece connected to an end of a belt extending in a longitudinal direction and a hook member configured to be selectively connected to the tongue piece by engaging a first lateral bar of the tongue piece in a hook opening defined by a hook portion thereof. A longitudinal dimension between the free end of the tongue piece and a base end side of the first lateral bar is substantially equal to or smaller than a width of the entrance of the hook opening, and the effective thickness of the first lateral bar is greater than the width of the entrance of the hook opening. Thus, the first lateral bar is held inside the hook opening as long as the tongue piece is kept in a flat position in a reliable manner. In particular, the first lateral bar can be freely introduced into the hook opening simply by raising the base end of the tongue piece while the first lateral bar is applied to the entrance of the hook opening so that the belt buckle device can be readily fastened even when the belt buckle device cannot be viewed from a proper viewing angle. Also, the belt buckle device can be readily fastened with a minimum dexterity requirement and very quickly.

Therefore, the existing technologies have drawbacks and deficiencies and need to be further improved and developed.

### SUMMARY

In view of the drawbacks of the existing technologies, the present disclosure aims to provide a magnetic buckle structure for a knee-joint brace and a knee-joint brace having the same, with the purpose of solving the problems in the existing technologies that the knee-joint brace is inconvenient to wear and remove.

The present disclosure solves the technological problems employing the following technical solution. A magnetic buckle structure for a knee-joint brace includes:
a joint brace body;
a lower magnetic buckle arranged on the joint brace body; and
an upper magnetic buckle arranged on the lower magnetic buckle and magnetically connected to the lower magnetic buckle.

Further, the lower magnetic buckle includes:
a lower magnetic buckle body integrally formed with the joint brace body;
an upper magnetic buckle connection end arranged on an upper end surface of the lower magnetic buckle body along a height direction; and
a lower magnet fixing end arranged on a lower end surface of the lower magnetic buckle body along the height direction.

Further, the upper magnetic buckle connection end includes:
an upper magnetic buckle fitting part arranged on the upper end surface of the lower magnetic buckle body along the height direction; and
a transverse baffle wall arranged on the upper magnetic buckle fitting part, and the upper magnetic buckle fitting part including a first groove, a first limit step and a second groove that are arranged in sequence;
a first vertical baffle wall arranged on one end of the transverse baffle wall;
a second vertical baffle wall arranged on the other end of the transverse baffle wall and symmetrical to the first vertical baffle wall; wherein
the first vertical baffle wall and the second vertical baffle wall are gradually increased in height along one end close to the transverse baffle wall.

Further, one ends of the first vertical baffle wall and the second vertical baffle wall away from the transverse baffle wall are both provided with a limit buckle, and the limit buckle includes:
a main body clamping bar arranged angled with the upper magnetic buckle fitting part and inclined toward the transverse baffle wall; and
a limit clamping tooth arranged on one end of the main body clamping bar away from the upper magnetic buckle fitting part and facing the transverse baffle wall.

Further, the lower magnet fixing end includes a lower magnet fixing groove and a plurality of enforcement ribs arranged on the periphery of the lower magnet fixing groove; and a magnet is arranged inside the magnet fixing groove.

Further, the upper magnetic buckle includes:
an upper magnetic buckle body;
an upper magnet fixing chamber arranged on the upper magnetic buckle body;
a buckling limit part arranged at an area of the upper magnetic buckle body corresponding to the limit clamping tooth; and
an assembly limit part arranged at an area of the upper magnetic buckle body close to the first groove.

Further, an edge of the upper magnetic buckle body corresponding to the first vertical baffle wall and the second vertical baffle wall, on one side along a height direction, is arranged as an arc shape.

Further, the buckling limit part includes:
a main body clamping bar receding section clamped on the main body clamping bar; and
a limit baffle clamped on the limit clamping tooth.

Further, the assembly limit part is arranged as a convex plate structure that is clamped to the second groove.

The present disclosure solves the technical problems employing another technical solution as follows. A knee-joint brace includes the magnetic buckle structure for the knee-joint brace described above.

The present disclosure provides a magnetic buckle structure for a knee-joint brace and a knee-joint brace having the same. The magnetic buckle structure for the knee-joint brace includes a joint brace body, a lower magnetic buckle arranged on the joint brace body, and an upper magnetic buckle arranged on the lower magnetic buckle and magnetically connected to the lower magnetic buckle. It is understandable that through the arrangement of the upper magnetic buckle and the lower magnetic buckle, a bandage of the knee-joint brace can be magnetically fixed or replaced, whereby being convenient for a user to wear and remove the knee-joint brace.

### BRIEF DESCRIPTION OF THE DRAWINGS

FIG. 1 is a perspective structure diagram of a magnetic buckle structure for a knee-joint brace according to the present disclosure.
FIG. 2 is a perspective exploded diagram of a magnetic buckle structure for a knee-joint brace according to the present disclosure.
FIG. 3 is a perspective exploded diagram of a magnetic buckle structure for a knee-joint brace according to the present disclosure from another angle of view.

Designators on the drawings are as described below.
10, a magnetic buckle structure for a knee-joint brace; 20, a joint brace body; 30, a lower magnetic buckle; 40, an upper magnetic buckle; 50, an S-pole magnet; 60, an N-pole magnet; 31, a lower magnetic buckle body; 32, an upper magnetic buckle connection end; 33, a lower magnet fixing end; 321, an upper magnetic buckle fitting part; 322, a transverse baffle wall; 323, a first vertical baffle wall; 324, a second vertical baffle wall; 325, a limit buckle; 3211, a first groove; 3212, a first limit step; 3213, a second groove; 3251, a main body clamping bar; 3252, a limit clamping tooth; 331, a lower magnet fixing groove; 332, a enforcement rib; 41, an upper magnetic buckle body; 42, an upper magnet fixing chamber; 43, a buckling limit part; 44, an assembly limit part; 45, a bandage spacing; 431, a main body clamping bar receding section; and 432, a limit baffle.

### DETAILED DESCRIPTION OF THE EMBODIMENTS

To make the purpose, the technical solution and the advantages of the present disclosure better understood, the present disclosure is described below in further detail in conjunction with specific embodiments by reference to the drawings. It should be understood that the specific embodiments described below are merely to illustrate, but to limit, the present disclosure.

In the description of the present disclosure, it is to be understood that the orientation or position relations indicated by such terms as "central", "longitudinal", "transverse", "above", "below", "front", "rear", "left", "right", "vertical", "horizontal", "top", "bottom", "inside", "outside", etc. are based on the orientation or position relations shown in the drawings, and are merely for conveniently describing the present disclosure and simplifying the description, rather than indicating or implying that the device or element must have the specific orientation and be constructed and operated according to the specific orientation. Therefore, they should not be construed as a limitation on the present disclosure. In addition, terms "first", "second" are merely for the purpose of description, but should not be understood as the indication or implication of relative importance or as the implicit indication of the quantity of the designated technical features. Therefore, features defined by "first" and "second" may specifically or implicitly include one or more such features. In the description of the present disclosure, unless otherwise stated, "a plurality of" means two or more than two.

In the description of the present disclosure, unless otherwise specifically stated and defined, terms such as "mounted", "interconnected", "connected", etc. should be interpreted expansively. For example, they may be fixed connection, also may be detachable connection, or integration; may be mechanical connection, also may be electrical connection; may be direct connection, also may be indirect connection through an intermediate, and may be internal communication between two elements. The ordinary skill in this field can understand the specific implication of the above terms in the present disclosure in accordance with specific conditions.

Knee joint is one of the joints in the human body that has a high load-bearing capacity and a complex structure. Knee joint diseases often occur in daily life, leading to limited human movement and seriously reducing the quality of life. In order to alleviate the damage of the knee joint diseases to the human body and improve the quality of life, a knee-joint brace is provided in the existing technologies, with which a support structure (such as a support arm) is arranged on the periphery of the knee joint to assist in supporting the knee joint associated bones, whereby assisting in fixing and supporting the knee joint. Therefore, the knee-joint brace can provide great protection and support for those people with knee joint discomfort, can reduce the pain and discomfort of the knee joints, and can provide walking support to certain extent for those people with walking disabilities, thereby reducing the condition of limited human movement and improving the quality of their lives. However, for most of those patients with knee-joint diseases, their self-care abilities are greatly limited, and they often have problems in wearing and removing the knee-joint braces. The present disclosure provides a magnetic buckle structure for a knee-joint brace and a knee-joint brace having the same in view of the problems in the existing technologies that the knee-joint brace is inconvenient to wear and remove. Through the arrangement of the upper magnetic buckle and the lower magnetic buckle, a bandage of the knee-joint brace can be magnetically fixed or replaced, whereby being convenient for a user to wear and remove the knee-joint brace. The following embodiments can be referred to for specific details.

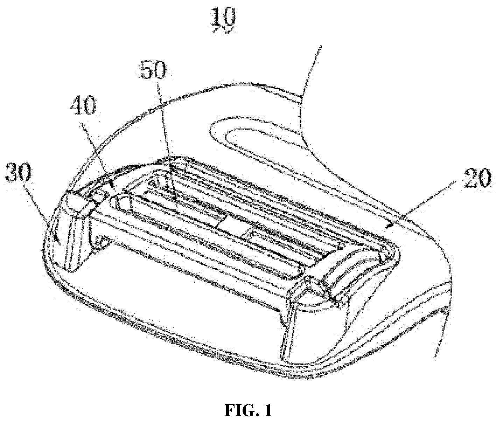

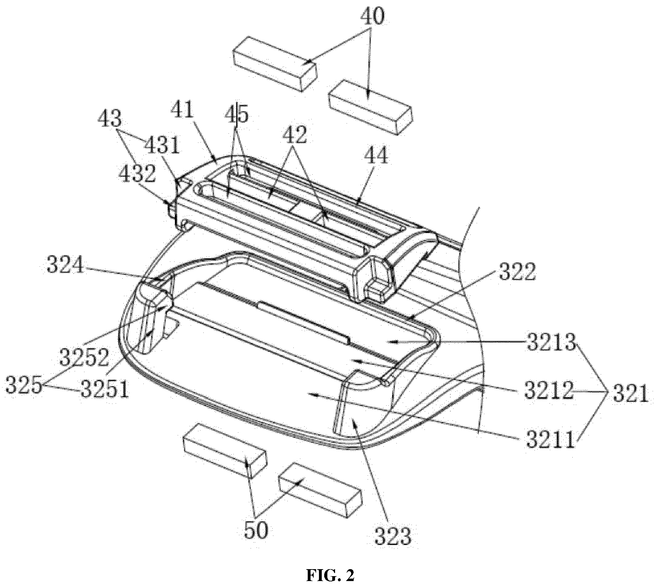

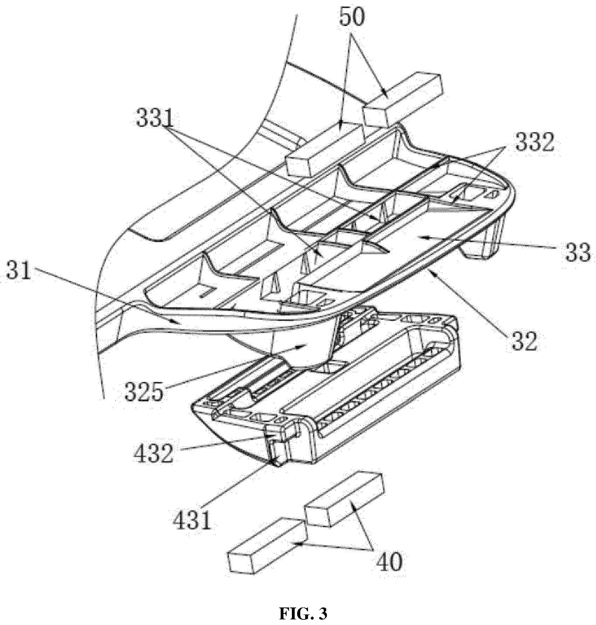

Referring to FIG. 1 to FIG. 3, a first embodiment of the present disclosure provides a magnetic buckle structure 10 for a knee-joint brace, including a joint brace body 20, a lower magnetic buckle 30 and an upper magnetic buckle 40. The lower magnetic buckle 30 is arranged on the joint brace body 20. The upper magnetic buckle 40 is arranged on the lower magnetic buckle 30 and magnetically connected to the lower magnetic buckle 30.

It is understandable that through the arrangement of the upper magnetic buckle 40 and the lower magnetic buckle 30 on the joint brace body, the knee-joint brace body 20 can be fixed with the assistance of the magnetic buckles. When the upper magnetic buckle 40 and the lower magnetic buckle 30 are magnetically connected, the knee-joint brace body 20 is preliminarily fixed, then the knee-joint brace can be tied or tightened conveniently. When to remove the knee-joint brace, the upper magnetic buckle 40 is separated from the lower magnetic buckle 30 before untying the knee-joint brace. It is greatly convenient to wear and remove the knee-joint brace. It is to be noted that the lower magnetic buckle 30 is integrally formed with the joint brace body 20, that is, the lower magnetic buckle 30 is a fixed magnetic buckle, while the upper magnetic buckle 40 is a moveable magnetic buckle.

In some other embodiments, the lower magnetic buckle 30 includes: a lower magnetic buckle body 31, an upper magnetic buckle connection end 32 and a lower magnet fixing end 33. The lower magnetic buckle body 31 is integrally formed with the joint brace body 20. The upper magnetic buckle connection end 32 is arranged on an upper end surface of the lower magnetic buckle body 31 along a height direction. The lower magnet fixing end 33 is arranged on a lower end surface of the lower magnetic buckle body 31 along a height direction.

It is understandable that the lower magnetic buckle body 31 is integrally formed with the joint brace body 20, thus guaranteeing the fastening function between the lower magnetic buckle 30 and the upper magnetic buckle 40. By arranging the magnet fixing end and the upper magnetic buckle connection end 32 on two end surfaces of the lower magnetic buckle body 31 respectively, a user can operate the magnetic buckle structure 10 of the knee-joint brace more conveniently.

In some other embodiments, the upper magnetic buckle connection end 32 includes: an upper magnetic buckle fitting part 321, a transverse baffle wall 322, a first vertical baffle wall 323 and a second vertical baffle wall 324. The upper magnetic buckle fitting part 321 is arranged on an upper end surface of the lower magnetic buckle body 31 along a height direction. The transverse baffle wall 322 is arranged on the upper magnetic buckle fitting part 321, and the upper magnetic buckle fitting part 321 includes a first groove 3211, a first limit step 3212 and a second groove 3213 that are arranged in sequence. The first vertical baffle wall 323 is arranged on one end of the transverse baffle wall 322. The second vertical baffle wall 324 is arranged on the other end of the transverse baffle wall 322 and symmetrical to the first vertical baffle wall 323. Herein, the first vertical baffle wall 323 and the second vertical baffle wall 324 are gradually increased in height along one end close to the transverse baffle wall 322.

It is understandable that the transverse baffle wall 322, the first vertical baffle wall 323 and the second vertical baffle wall 324 are combined to limit the upper magnetic buckle 40. When the upper magnetic buckle 40 and the lower magnetic buckle 30 are magnetically connected, the upper magnetic buckle 40 is adsorbed onto the upper magnetic buckle fitting part 321. Further, the transverse baffle wall 322, the first vertical baffle wall 323 and the second vertical baffle wall 324 are arranged as a U shape.

In some other embodiments, one ends of the first vertical baffle wall 323 and the second vertical baffle wall 324 away from the transverse baffle wall 322 are both provided with a limit buckle 325, and the limit buckle 325 includes: a main body clamping bar 3251 and a limit clamping tooth 3252. The main body clamping bar 3251 is arranged angled with the upper magnetic buckle fitting part 321 and inclined toward the transverse baffle wall 322. The limit clamping tooth 3252 is arranged on one end of the main body clamping bar 3251 away from the upper magnetic buckle fitting part 321 and faces the transverse baffle wall 322.

It is understandable that one ends of the first vertical baffle wall 323 and the second vertical baffle wall 324 away from the transverse baffle wall 322 are both provided with a limit buckle 325, through which limit buckle 325 to clamp and fix the upper magnetic buckle 40. The main body clamping bar 3251 and the limit clamping tooth 3252 are arranged as an L shape. By arranging the main body clamping bar 3251 to be angled with the upper magnetic buckle fitting part 321 and inclined toward the transverse baffle wall 322, the capability of the limit buckle 325 is improved in limiting and fixing the upper magnetic buckle 40.

In some other embodiments, the lower magnet fixing end 33 includes a lower magnet fixing groove 331 and a plurality of enforcement ribs 332 arranged on the periphery of the lower magnet fixing groove 331. A magnet is arranged inside the magnet fixing groove.

It is understandable that the lower magnetic buckle 30 is arranged as a fixed magnet structure, and through the arrangement of the plurality of enforcement ribs 332, the structure strength of the lower magnet body can be improved.

In some other embodiments, the upper magnetic buckle 40 includes: an upper magnetic buckle body 41, an upper magnet fixing chamber 42, a buckling limit part 43 and an assembly limit part 44. The upper magnet fixing chamber 42 is arranged on the upper magnetic buckle body 41. The buckling limit part 43 is arranged at an area of the upper magnetic buckle body 41 corresponding to the limit clamping tooth 3252. The assembly limit part 44 is arranged at an area of the upper magnetic buckle body 41 close to the first groove 3211.

It is understandable that through the upper magnet fixing chamber 42 arranged on the upper magnetic buckle body 41, a magnet can be effectively fixed and mounted, so that the upper magnetic buckle 40 possesses a magnetic attraction force. The buckling limit part 43 is arranged to fit with the limit buckle 325, whereby clamping and fixing the upper magnetic buckle 40 and the lower magnetic buckle 30, guaranteeing the connection strength of the magnetic buckle structure 10 for the knee-joint brace. The assembly limit part is arranged to fit with the first groove 3211, whereby reducing the assembly thickness between the upper magnetic buckle 40 and the lower magnetic buckle 30. It is to be noted that when the buckling limit part 43 is clamped on the limit buckle 325, the area of the upper magnetic buckle body 41 close to the buckling limit part 43 is received in the second groove 3213, further reducing the assembly thickness between the upper magnetic buckle 40 and the lower magnetic buckle 30 and enhancing the connection strength between the upper magnetic buckle 40 and the lower magnetic buckle 30.

In some other embodiments, an edge of the upper magnetic buckle body 41 corresponding to the first vertical baffle wall 323 and the second vertical baffle wall 324, on one side along a height direction, is arranged as an arc shape.

It is understandable that by controlling an edge of the upper magnetic buckle body 41 corresponding to the first vertical baffle wall 323 and the second vertical baffle wall 324, on one side along a height direction, to be arranged as an arc shape, the buckling limit part 43 can be conveniently rotated into the limit buckle 325, whereby the upper magnetic buckle 40 can be conveniently assembled into the lower magnetic buckle 30.

In some other embodiments, the buckling limit part 43 includes: a main body clamping bar receding section 431 and a limit baffle 432. The main body clamping bar receding section 431 is clamped on the main body clamping bar 3251. The limit baffle 432 is clamped on the limit clamping tooth 3252.

It is understandable that the limit baffle 432 and the limit clamping tooth 3252 assist in restricting the upper magnetic buckle 40 and the lower magnetic buckle 30 from disengaging along the height direction, guaranteeing the connection strength between the upper magnetic buckle 40 and the lower magnetic buckle 30.

In some other embodiments, the assembly limit part 44 is arranged as a convex plate structure that is clamped to the second groove 3213.

It is understandable that the assembly limit part 44 is arranged as a convex plate structure so that the assembly limit part 44 can achieve some auxiliary fixation while being fitted into the second groove 3213.

In some specific embodiments, an S-pole magnet 50 is arranged inside the upper magnetic buckle 40 while an N-pole magnet 60 is arranged inside the lower magnetic buckle 30, or, an N-pole magnet 60 is arranged inside the upper magnetic buckle 40 while an S-pole magnet 50 is arranged inside the lower magnetic buckle 30. The magnets in both the upper magnetic buckle 40 and the lower magnetic buckle 30 are fixed by gluing. The upper magnetic buckle body 41 is formed with bandage spacings 45 between the upper magnet fixing chamber 42, the buckling limit part 43 and the assembly limit part 44. A bandage can be arranged in the bandage spacings 45, through which bandage the upper magnetic buckle 40 can be lifted up, so that the upper magnetic buckle 40 is separated from the lower magnetic buckle 30, whereby facilitating the removal of the knee-joint brace.

A second embodiment of the present disclosure provides a knee-joint brace, including the magnetic buckle structure 10 for the knee-joint brace described in the first embodiment of the present disclosure. It is understandable that by employing the magnetic buckle structure 10 for the knee-joint brace provided in the first embodiment of the present disclosure, the knee-joint brace provided in the present embodiment can magnetically fix or replace the bandage of the knee-joint brace due to the arrangement of the upper magnetic buckle 40 and the lower magnetic buckle 30, whereby being convenient for a user to wear and remove the knee-joint brace.

To sum up, the present disclosure provides a magnetic buckle structure for a knee-joint brace and a knee-joint brace having the same. The magnetic buckle structure for the knee-joint brace includes a joint brace body, a lower magnetic buckle arranged on the joint brace body, and an upper magnetic buckle arranged on the lower magnetic buckle and magnetically connected to the lower magnetic buckle. It is understandable that through the arrangement of the upper magnetic buckle and the lower magnetic buckle, a bandage of the knee-joint brace can be magnetically fixed or replaced, whereby being convenient for a user to wear and remove the knee-joint brace.

It should be understood that the application of the present disclosure is not limited to the above examples. For the ordinary staff in this field, improvements or variations may be made according to the above description, and these improvements or variations are intended to be included within the scope of protection of the claims appended hereinafter.

## Claims

1. A magnetic buckle structure for a knee-joint brace, comprising:
a joint brace body (20);
a lower magnetic buckle (30) adapted to be arranged on the joint brace body (20); and
an upper magnetic buckle (40) adapted to be arranged on the lower magnetic buckle (30) and magnetically connected to the lower magnetic buckle (30),
wherein the lower magnetic buckle (30) comprises:
a lower magnetic buckle body (31) integrally formed with the joint brace body (20);
an upper magnetic buckle connection end (32) arranged on an upper end surface of the lower magnetic buckle body (31) along a height direction; and
a lower magnet fixing end (33) arranged on a lower end surface of the lower magnetic buckle body (31) along the height direction,
**characterized in**
**that** the upper magnetic buckle connection end (32) comprises:
an upper magnetic buckle fitting part (321) arranged on the upper end surface of the lower magnetic buckle body (30) along the height direction; and
a transverse baffle wall (322) arranged on the upper magnetic buckle fitting part (321), and the upper magnetic buckle fitting part (321) comprising a first groove (3211), a first limit step (3212) and a second groove (3213) that are arranged in sequence;
a first vertical baffle wall (323) arranged on one end of the transverse baffle wall (322);
a second vertical baffle wall (324) arranged on the other end of the transverse baffle wall (322) and symmetrical to the first vertical baffle wall (323); wherein
the first vertical baffle wall (323) and the second vertical baffle wall (324) are gradually increased in height along one end close to the transverse baffle wall (322).

2. The magnetic buckle structure for the knee-joint brace according to claim 1, wherein one ends of the first vertical baffle wall (323) and the second vertical baffle wall (324) away from the transverse baffle wall (322) are both provided with a limit buckle (325), and the limit buckle (325) comprises:
a main body clamping bar (3251) arranged angled with the upper magnetic buckle fitting part (321) and inclined toward the transverse baffle wall (322); and
a limit clamping tooth (3252) arranged on one end of the main body clamping bar (3251) away from the upper magnetic buckle fitting part (321) and facing the transverse baffle wall (322).

3. The magnetic buckle structure for the knee-joint brace according to claim 2, wherein the lower magnet fixing end (33) comprises a lower magnet fixing groove (331) and a plurality of enforcement ribs (332) arranged on the periphery of the lower magnet fixing groove (331); and a magnet is arranged inside the magnet fixing groove.

4. The magnetic buckle structure for the knee-joint brace according to claim 2, wherein the upper magnetic buckle (40) comprises:
an upper magnetic buckle body (41);
an upper magnet fixing chamber (42) arranged on the upper magnetic buckle body (41);
a buckling limit part (43) arranged at an area of the upper magnetic buckle body (41) corresponding to the limit clamping tooth (3252); and
an assembly limit part (44) arranged at an area of the upper magnetic buckle body (41) close to the first groove (3211).

5. The magnetic buckle structure for the knee-joint brace according to claim 4, wherein
an edge of the upper magnetic buckle body (41) corresponding to the first vertical baffle wall (323) and the second vertical baffle wall (324), on one side along a height direction, is arranged as an arc shape.

6. The magnetic buckle structure for the knee-joint brace according to claim 4, wherein the buckling limit part (43) comprises:
a main body clamping bar receding section (431) clamped on the main body clamping bar (3251); and
a limit baffle (432) clamped on the limit clamping tooth (3252).

7. The magnetic buckle structure for the knee-joint brace according to claim 4, wherein the assembly limit part (44) is arranged as a convex plate structure that is clamped to the second groove (3213).

8. A knee-joint brace, comprising the magnetic buckle structure for the knee-joint brace according to any one of claims 1 to 7.

## Patentansprüche

1. Magnetische Schnallenstruktur für eine Kniegelenkstrebe, umfassend:
einen Gelenkstrebenkörper (20);
eine untere magnetische Schnalle (30), die angepasst ist, um auf dem Gelenkstrebenkörper (20) angeordnet zu werden; und
eine obere magnetische Schnalle (40), die angepasst ist, um auf der unteren magnetischen Schnalle (30) angeordnet und magnetisch mit der unteren magnetischen Schnalle (30) verbunden zu werden,
wobei die untere magnetische Schnalle (30) Folgendes umfasst:
einen unteren magnetischen Schnallenkörper (31), der einstückig mit dem Gelenkstrebenkörper (20) ausgebildet ist;
ein oberes magnetisches Schnallenverbindungsende (32), das auf einer oberen Endfläche des unteren magnetischen Schnallenkörpers (31) entlang einer Höhenrichtung angeordnet ist; und
ein unteres magnetisches Befestigungsende (33), das auf einer unteren Endfläche des unteren magnetischen Schnallenkörpers (31) entlang der Höhenrichtung angeordnet ist,
**dadurch gekennzeichnet,**
**dass** das obere magnetische Schnallenverbindungsende (32) Folgendes umfasst:
einen oberen magnetischen Schnallenanschlussteil (321), der auf der oberen Endfläche des unteren magnetischen Schnallenkörpers (30) entlang der Höhenrichtung angeordnet ist; und
eine querverlaufende Prallwand (322), die auf dem oberen magnetischen Schnallenanschlussteil (321) angeordnet ist, wobei der obere magnetische Schnallenanschlussteil (321) eine erste Nut (3211), eine erste Begrenzungsstufe (3212) und eine zweite Nut (3213) umfasst, die nacheinander angeordnet sind;
eine erste vertikale Prallwand (323), die auf einem Ende der querverlaufenden Prallwand (322) angeordnet ist;
eine zweite vertikale Prallwand (324), die auf dem anderen Ende der querverlaufenden Prallwand (322) und symmetrisch zur ersten vertikalen Prallwand (323) angeordnet ist; wobei
die erste vertikale Prallwand (323) und die zweite vertikale Prallwand (324) entlang eines Endes nahe der querverlaufenden Prallwand (322) allmählich in der Höhe zunehmen.

2. Magnetische Schnallenstruktur für die Kniegelenkstrebe nach Anspruch 1, wobei ein Ende der ersten vertikalen Prallwand (323) und der zweiten vertikalen Prallwand (324), die von der querverlaufenden Prallwand (322) entfernt sind, beide mit einer Begrenzungsschnalle (325) versehen sind, wobei die Begrenzungsschnalle (325) Folgendes umfasst:
eine Hauptkörperklemmleiste (3251), die mit dem oberen magnetischen Schnallenanschlussteil (321) abgewinkelt und in Richtung der querverlaufenden Prallwand (322) geneigt angeordnet ist; und
einen Begrenzungsklemmzahn (3252), der auf einem Ende der Hauptkörperklemmleiste (3251) entfernt von dem oberen magnetischen Schnallenanschlussteil (321) angeordnet ist und der querverlaufenden Prallwand (322) gegenüberliegt.

3. Magnetische Schnallenstruktur für die Kniegelenkstrebe nach Anspruch 2, wobei das untere magnetische Befestigungsende (33) eine untere magnetische Befestigungsnut (331) und eine Vielzahl von Verstärkungsrippen (332) umfasst, die am Umfang der unteren magnetischen Befestigungsnut (331) angeordnet sind; wobei ein Magnet innerhalb der magnetischen Befestigungsnut angeordnet ist.

4. Magnetische Schnallenstruktur für die Kniegelenkstrebe nach Anspruch 2, wobei die obere magnetische Schnalle (40) Folgendes umfasst:
einen oberen magnetischen Schnallenkörper (41);
eine obere magnetische Befestigungskammer (42), die auf dem oberen magnetischen Schnallenkörper (41) angeordnet ist;
einen Schnallenbegrenzungsteil (43), der in einem Bereich des oberen magnetischen Schnallenkörpers (41) angeordnet ist, der dem Begrenzungsklemmzahn (3252) entspricht; und
einen Montagebegrenzungsteil (44), der in einem Bereich des oberen magnetischen Schnallenkörpers (41) nahe der ersten Nut (3211) angeordnet ist.

5. Magnetische Schnallenstruktur für die Kniegelenkstrebe nach Anspruch 4, wobei
eine Kante des oberen magnetischen Schnallenkörpers (41), die der ersten vertikalen Prallwand (323) und der zweiten vertikalen Prallwand (324) entspricht, auf einer Seite entlang einer Höhenrichtung als eine Bogenform angeordnet ist.

6. Magnetische Schnallenstruktur für die Kniegelenkstrebe nach Anspruch 4, wobei der Schnallenbegrenzungsteil (43) Folgendes umfasst:
einen Hauptkörperklemmleisten-Rückzugsabschnitt (431), der auf der Hauptkörperklemmleiste (3251) geklemmt ist; und
eine Begrenzungsprallplatte (432), die auf dem Begrenzungsklemmzahn (3252) geklemmt ist.

7. Magnetische Schnallenstruktur für die Kniegelenkstrebe nach Anspruch 4, wobei der Montagebegrenzungsteil (44) als eine konvexe Plattenstruktur angeordnet ist, die in der zweiten Nut (3213) geklemmt wird.

8. Kniegelenkstrebe, umfassend die Magnetschnallenstruktur für die Kniegelenkstrebe nach einem der Ansprüche 1 bis 7.

## Revendications

1. Structure de boucle magnétique pour une orthèse d'articulation de genou, comprenant :
un corps d'orthèse d'articulation (20) ;
une boucle magnétique inférieure (30) conçue pour être disposée sur le corps d'orthèse d'articulation (20) ; et
une boucle magnétique supérieure (40) conçue pour être disposée sur la boucle magnétique inférieure (30) et reliée magnétiquement à la boucle magnétique inférieure (30),
dans laquelle la boucle magnétique inférieure (30) comprend :
un corps de boucle magnétique inférieure (31) formé intégralement avec le corps d'orthèse d'articulation (20) ;
une extrémité de liaison de boucle magnétique supérieure (32) disposée sur une surface d'extrémité supérieure du corps de boucle magnétique inférieure (31) le long d'une direction de hauteur ; et
une extrémité de fixation d'aimant inférieur (33) disposée sur une surface d'extrémité inférieure du corps de boucle magnétique inférieure (31) le long de la direction de hauteur,
**caractérisée en ce que**
l'extrémité de liaison de boucle magnétique supérieure (32) comprend :
une partie d'ajustement de boucle magnétique supérieure (321) disposée sur la surface d'extrémité supérieure du corps de boucle magnétique inférieure (30) dans la direction de hauteur ; et
une paroi déflectrice transversale (322) disposée sur la partie d'ajustement de boucle magnétique supérieure (321), et la partie d'ajustement de boucle magnétique supérieure (321) comprenant une première rainure (3211), une première marche de limitation (3212) et une seconde rainure (3213) qui sont disposées en séquence ;
une première paroi déflectrice verticale (323) disposée sur une extrémité de la paroi déflectrice transversale (322) ;
une seconde paroi déflectrice verticale (324) disposée sur l'autre extrémité de la paroi déflectrice transversale (322) et symétriquement par rapport à la première paroi déflectrice verticale (323) ; dans laquelle
la première paroi déflectrice verticale (323) et la seconde paroi déflectrice verticale (324) augmentent progressivement en hauteur le long d'une extrémité proche de la paroi déflectrice transversale (322).

2. Structure de boucle magnétique pour l'orthèse d'articulation de genou selon la revendication 1, dans laquelle une extrémité de la première paroi déflectrice verticale (323) et de la seconde paroi déflectrice verticale (324) opposée à la paroi déflectrice transversale (322) est pourvue d'une boucle de limitation (325), et la boucle de limitation (325) comprend :
une barre de serrage de corps principal (3251) disposée en angle avec la partie d'ajustement de boucle magnétique supérieure (321) et de façon inclinée vers la paroi déflectrice transversale (322) ; et
une dent de serrage de limitation (3252) disposée sur une extrémité de la barre de serrage de corps principal (3251) opposée à la partie d'ajustement de boucle magnétique supérieure (321) et faisant face à la paroi déflectrice transversale (322).

3. Structure de boucle magnétique pour l'orthèse d'articulation de genou selon la revendication 2, dans laquelle l'extrémité de fixation d'aimant inférieur (33) comprend une rainure de fixation d'aimant inférieur (331) et une pluralité de nervures d'exécution (332) disposées sur la périphérie de la rainure de fixation d'aimant inférieur (331) ; et un aimant est disposé à l'intérieur de la rainure de fixation d'aimant.

4. Structure de boucle magnétique pour l'orthèse d'articulation de genou selon la revendication 2, dans laquelle la boucle magnétique supérieure (40) comprend :
un corps de boucle magnétique supérieure (41) ;
une chambre de fixation d'aimant supérieur (42) disposée sur le corps de boucle magnétique supérieure (41) ;
une partie de limitation de bouclage (43) disposée au niveau d'une zone du corps de boucle magnétique supérieure (41) correspondant à la dent de serrage de limitation (3252) ; et
une partie de limitation d'assemblage (44) disposée au niveau d'une zone du corps de boucle magnétique supérieure (41) proche de la première rainure (3211).

5. Structure de boucle magnétique pour l'orthèse d'articulation de genou selon la revendication 4, dans laquelle
un bord du corps de boucle magnétique supérieure (41) correspondant à la première paroi déflectrice verticale (323) et à la seconde paroi déflectrice verticale (324), sur un côté le long d'une direction de hauteur, est disposé en forme d'arc.

6. Structure de boucle magnétique pour l'orthèse d'articulation de genou selon la revendication 4, dans laquelle la partie de limitation de bouclage (43) comprend :
une section de recul de barre de serrage de corps principal (431) serrée sur la barre de serrage de corps principal (3251) ; et
un déflecteur de limitation (432) serré sur la dent de serrage de limitation (3252).

7. Structure de boucle magnétique pour l'orthèse d'articulation de genou selon la revendication 4, dans laquelle la partie de limitation d'assemblage (44) est disposée comme une structure de plaque convexe qui est serrée sur la seconde rainure (3213).

8. Orthèse d'articulation de genou comprenant la structure de boucle magnétique pour l'orthèse d'articulation de genou selon l'une quelconque des revendications 1 à 7.
